(19) Europäisches Patentamt
European Patent Office
Office européen des brevets

(11) Veröffentlichungsnummer : **0 400 509 B1**

(12) # EUROPÄISCHE PATENTSCHRIFT

(45) Veröffentlichungstag der Patentschrift : **07.04.93 Patentblatt 93/14**

(51) Int. Cl.$^5$ : **C07C 49/385,** C07C 49/203, C07C 45/67

(21) Anmeldenummer : **90110008.1**

(22) Anmeldetag : **26.05.90**

(54) **Verfahren zur Herstellung von Muscon, Zwischenprodukte für dieses Verfahren sowie deren Herstellung.**

(30) Priorität : **02.06.89 DE 3918015**

(43) Veröffentlichungstag der Anmeldung : **05.12.90 Patentblatt 90/49**

(45) Bekanntmachung des Hinweises auf die Patenterteilung : **07.04.93 Patentblatt 93/14**

(84) Benannte Vertragsstaaten : **CH DE FR GB LI NL**

(56) Entgegenhaltungen :
**EP-A- 0 012 390**
**EP-A- 0 015 412**
**EP-A- 0 230 499**
**AT-B- 108 150**
**AT-B- 230 347**
**DE-A- 2 453 660**
**DE-C- 875 351**
**US-A- 3 718 696**
**PATENT ABSTRACTS OF JAPAN, unexamined applications, C Field, Band 9, Nr. 66, 26. März 1985; THE PATENT OFFICE JAPANESE GOVERNMENT Seite 99 C 271**

(73) Patentinhaber : **BASF Aktiengesellschaft**
**Carl-Bosch-Strasse 38**
**W-6700 Ludwigshafen (DE)**

(72) Erfinder : **Huellmann, Michael, Dr.**
**Siegfriedstrasse 41**
**W-6148 Heppenheim (DE)**
Erfinder : **Kuekenhoehner, Thomas, Dr.**
**Seidelstrasse 2**
**W-6710 Frankenthal (DE)**
Erfinder : **Brenner, Karl**
**Riedsaumstrasse 40**
**W-6700 Ludwigshafen (DE)**
Erfinder : **Becker, Rainer, Dr.**
**Im Haseneck 22**
**W-6702 Bad Duerkheim (DE)**
Erfinder : **Irgang, Matthias, Dr.**
**Andreas-Hofer-Weg 41**
**W-6900 Heidelberg (DE)**
Erfinder : **Schommer, Charles, Dr.**
**Moerikestrasse 32**
**W-6700 Ludwigshafen (DE)**

EP 0 400 509 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung des begehrten Riechstoffs Muscon durch intramolekulare Aldolkondensation von Hexadecan-2,15-dion oder Hexadecadien-2,15-dionen in der Gasphase sowie neue Hexadecadien-2,15-dione und ein vorteilhaftes Verfahren zu deren Herstellung.

Muscon (3-Methyl-cyclopentadecanon) der Formel I

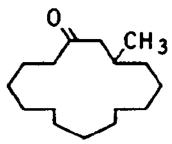

(I)

ist einer der wichtigsten Inhaltsstoffe der in der Parfümerie sehr begehrten natürlichen Moschusextrakte. Infolge des extrem hohen Preises für natürliche Extrakte ist die synthetische Herstellung von I von großem Interesse, insbesondere, da I allen anderen bekannten Moschusriechstoffen, wie den Tetralin- oder Nitromoschusverbindungen, weit überlegen ist.

Die bisher angewendeten Herstellungsmethoden basieren überwiegend auf Ringerweiterungsreaktionen, ausgehend von Cyclododekanon (vgl. beispielsweise Helv. Chim. Acta 71 (1988), S. 1704 - 1718 und in loc. Cit. genannte Literatur). Diese Methoden weisen alle teilweise recht aufwendige mehrstufige Verfahrensschritte auf und sind daher für eine wirtschaftliche Nutzung unattraktiv.

Andere bekannte Synthesemethoden beinhalten intramolekulare Kondensationsreaktionen wie Aldol-, Dieckmann- oder Acyloinkondensation (siehe dazu Houben-Weyl, Methoden der organischen Chemie, Bd. 4/2, S. 729 - 815). Diese Methoden haben alle den großen Nachteil, daß nur in sehr hoher Verdünnung relativ gute Ausbeuten an Makrocyclen erhalten werden.

In Helv. Chim. Acta, 62 (1979), Seiten 2673 - 2680 wird ein neuartiges Syntheseverfahren für Muscon auf Basis 4,8-Dodecadiendiol vorgestellt. Schlüsselschritt ist hierbei die säurekatalysierte intramolekulare Cyclisierung eines offenkettigen Hydroxyacetals zum bicyclischen Dihydropyran, wobei allerdings wegen des notwendigen Verdünnungsprinzips hohe Lösungsmittelmengen erforderlich sind, wodurch dieses Verfahren nur für die Synthese von Labormengen sinnvoll einsetzbar ist.

Eine an sich recht gute Möglichkeit für die Herstellung von I schien die erstmals von Stoll (vgl. Helv. Chim. Acta, 30 (1947), Seiten 2019-2023) beschriebene Aldolkondensation ausgehend von Hexadecan-2,15-dion der Formel IIa zu sein,

$$CH_3\text{-}CO\text{-}(CH_2)_{12}\text{-}CO\text{-}CH_3 \qquad \text{(IIa)}$$

da hierbei die Methylgruppe in 3-Stellung von I gleich mit eingebracht wird.

Allerdings war dieses Verfahren mit beachtlichen Nachteilen behaftet:

1) Waren die Herstellmöglichkeiten für das als Ausgangsmaterial benötigte Keton der Formel IIa bzw. von adäquaten 2,15-Diketonen bislang unbefriedigend.

2) Sind die bei der Aldolkondensation zu erzielenden Ausbeuten trotz des Arbeitens in stark verdünnten Lösungen relativ gering (gemäß loc. cit. 17 %).

Zu 1:

Es hat daher in der Vergangenheit nicht an Versuchen gefehlt, geeignete Herstellmöglichkeiten für IIa zu entwickeln. Stoll synthetisierte es nach folgendem Reaktionsschema:

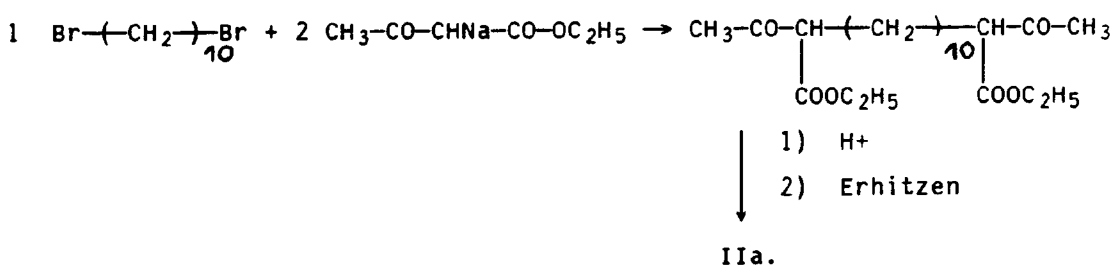


Nachteilig an dieser Synthese ist insbesondere die Verwendung des kostspieligen und außerdem toxikologisch bedenklichen 1,10-Dibromdecans.

Ferner wurde in J. Am. Chem. Soc. 82 (1960), Seiten 1447 - 1450, die folgende Synthese ausgehend von 2,2′,5′,2″-Terthienyl vorgestellt:

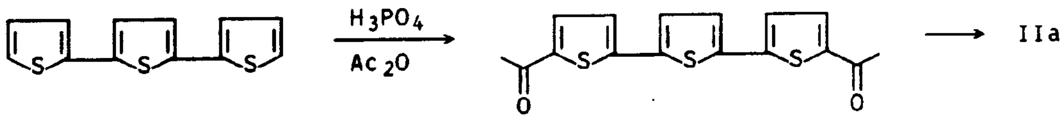


Zur Synthese größerer Diketonmengen ist diese Synthese jedoch wegen der schlechten Zugänglichkeit des Ausgangsmaterials nicht geeignet.

Zwei weitere Verfahren zur Herstellung des Diketons IIa jeweils ausgehend von Butadien wurden von Tsuji et al. beschrieben:

a) in Chem. Lett. 1976, Seiten 773-774:

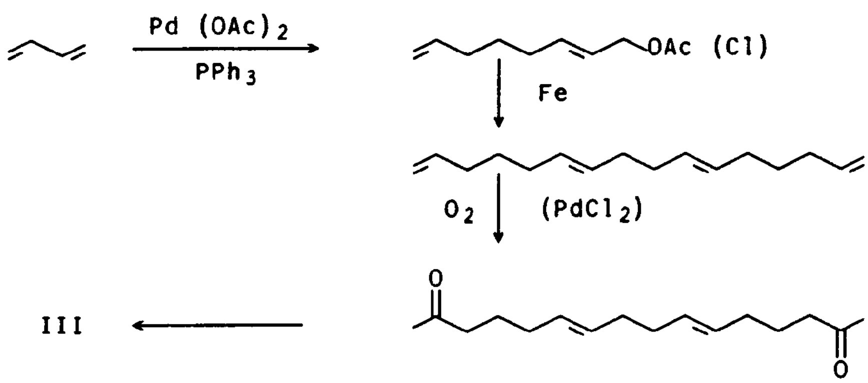


und

b) in Bull. Chem. Soc. Japan, 51 (1978), Seite 1915:

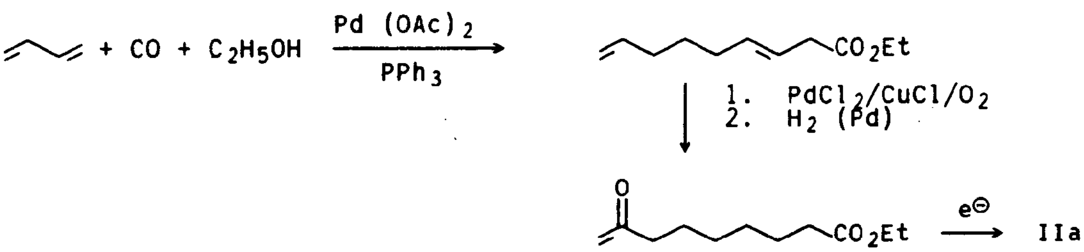


In beiden Verfahren kommen teure Palladiumkatalysatoren zum Einsatz, wodurch auch diese Synthesen für einen industriellen Einsatz unattraktiv werden.

Weiterhin ist aus Bull. Chem. Soc. Japan, 56 (1983) Seiten 345-346 ein Verfahren zur Herstellung von IIa ausgehend von α,ω-Tetradecandicarbonsäure bekannt. Nachteilig an diesem Verfahren ist die schlechte Zugänglichkeit der Ausgangsverbindung.

Aus J. Organomet. Chem. 264 (1984), Seiten 229-37 ist außerdem ein Verfahren zur Herstellung von IIa ausgehend von $(CH_3)_3Si\text{-}CH_2\text{-}CH{=}CH\text{-}CH_2\text{-}CH_2\text{-}C(CH_3){=}CH\text{-}CH_2\text{-}Si(CH_3)_3$ bekannt. Nachteilig an diesem Verfahren ist neben der schlechten Zugänglichkeit der Ausgangsverbindung die Notwendigkeit der Verwendung von problematischen Reagentien, wie dem leicht entzündlichen Kaliumhydrid.

Es war daher eine Aufgabe der Erfindung eine technisch einfache und billige Herstellweise für das Hexadecan-2,15-dion zu finden.

Diese Aufgabe wurde durch Bereitstellen der neuen 2-fach ungesättigten 2,15-Diketone der allgemeinen Formel II

$$CH_3\text{-}CO\text{-}X\text{-}CO\text{-}CH_3 \qquad (II),$$

in der X für einen unverzweigten Alkadienylenrest der Struktur

$$-CH{=}CH-(CH_2)_8-CH{=}CH- \quad (b),$$

$$-CH_2-CH{=}CH-(CH_2)_6-CH{=}CH-CH_2- \quad (c)$$

oder

$$-CH_2-CH_2-CH{=}CH-(CH_2)_4-CH{=}CH-CH_2-CH_2- \quad (d)$$

steht,

im wesentlichen gelöst, da diese 2,15-Diketone einerseits auf technisch einfache Weise herstellbar sind und andererseits recht vorteilhaft in das Diketon IIa sowie in Muscon überführt werden können.

Gegenstand der Erfindung ist daher auch ein Verfahren zur Herstellung eines Gemisches der 2-fach ungesättigten 2,15-Diketone der Formeln IIb und IIc

$$CH_3-CO-CH{=}CH-(CH_2)_8-CH{=}CH-CO-CH_3 \quad (IIb)$$

$$CH_3-CO-CH_2-CH{=}CH-(CH_2)_6-CH{=}CH-CH_2-CO-CH_3 \quad (IIc),$$

das dadurch gekennzeichnet ist, daß man 1,10-Decan-diol der Formel III

$$OH-CH_2-(CH_2)_8-CH_2-OH \quad (III)$$

oxidativ dehydriert,

das dabei erhaltene 1,10-Decan-dial der Formel IV

$$OHC-(CH_2)_8-CHO \quad (IV)$$

einer Wittig-Reaktion mit 2 Mol eines 2-Oxo-propenyl-triphenylphosphoniumsalzes der Formel V

$$CH_3-\overset{\overset{O}{\|}}{C}-C{=}\overset{\oplus}{P}\,(Phenyl)_3\ Y^{\ominus} \quad (V),$$

worin

$Y^{\ominus}$ für $Cl^-$, $Br^-$ oder $HSO_4^{\ominus}$ steht, unterwirft und das Reaktionsprodukt destilliert.

Das für dieses Verfahren benötigte 1,10-Decan-diol ist eine handelsübliche Verbindung, die durch alkalische Spaltung von Ricinusöl und anschließende Hydrierung der erhaltenen Sebacinsäure auf relativ einfache Weise erhalten werden kann.

Die oxidative Dehydrierung des 1,10-Decan-diols in das 1,10-Decandial kann auf verschiedene Weise realisiert werden. Mit Vorteil arbeitet man in der Gasphase an Silberkatalysatoren, wie beispielsweise in Manuf.Chem. Aerosol News 37 (1966) Seiten 42-45 für $C_5$-$C_{14}$-Alkohole beschrieben ist. Nähere Einzelheiten über die Reaktionsbedingungen für diese Verfahrensstufe können der genannten Literaturstelle entnommen werden. Sie kann aber auch durch Oxidation in Methylenchlorid mit wäßriger NaOCl-Lösung in Gegenwart von katalytischen Mengen von 4-Methoxy-2,2,6,6-tetramethyl-piperidin-N-oxid erfolgen. Bezüglich näherer Einzelheiten über diese Verfahrensstufe verweisen wir auf J. Org. Chem. 52 (1987), Seiten 2559-2562.

Zur Durchführung der Wittig-Reaktion mit einem 2-Oxy-propenyl-triphenylphosphoniumsalz der Formel V geht man mit Vorteil so vor, daß man das Triphenylphosphoniumsalz in einem inerten organischen Lösungsmittel, wie Methylenchlorid vorlegt und hierzu die Lösung des 1,10-Dials in einem inerten, vorzugsweise dem gleichen inerten Lösungsmittel langsam zufügt und das Reaktionsgemisch noch 1 bis 2 Stunden nachrührt.

Die Aufarbeitung erfolgt auf für Wittig-Reaktionen übliche Weise durch Einengen, Ausfällen des Phosphinoxids und Destillation. Durch $^{13}C$-NMR-Untersuchungen zeigte sich, daß bei der destillativen Aufarbeitung des Reaktionsgemisches ein Teil des zunächst gebildeten Diketons der Formel IIb zu dem Diketon der Formel IIc isomerisiert.

Gegenstand der Erfindung ist auch ein Verfahren zur Herstellung des 2-fach ungesättigten 2,15-Diketons der Formel IId

$$CH_3-CO-CH_2-CH_2-CH{=}CH-(CH_2)_4-CH{=}CH-CH_2-CH_2-CO-CH_3 \quad (IId)$$

das dadurch gekennzeichnet ist, daß man

A. 1,6-Hexan-diol der Formel VI

$HO-CH_2-(CH_2)_4-CH_2-OH$ (VI)

oxidativ dehydriert,

B. den dabei erhaltenen Adipindialdehyd der Formel VII

$OHC-(CH_2)_4-CHO$ (VII)

mit je 2 Mol eines Vinylmagnesiumhalogenids der Formel VIII

$CH_2=CH-Mg-Hal$ (VIII),

in der Hal für Cl oder Br steht, in einer Grignard-Reaktion umsetzt und

C. das dabei erhaltene 1,9-Decadien-3,8-diol der Formel IX

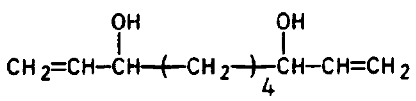

(IX)

einer Carroll-Reaktion mit einem Acetessigsäurealkylester unterwirft.

Das für dieses Verfahren benötigte 1,6-Hexan-diol ist eine großtechnisch verfügbare und daher billige Ausgangsverbindung.

Die oxidative Dehydrierung des 1,6-Hexan-diols zu Adipindialdehyd kann beispielsweise analog zu der oben beschriebenen oxidativen Dehydrierung des 1,10-Decandiols in der Gasphase an einem Silberkatalysator oder mit wäßriger NaOCl-Lösung in Gegenwart von 4-Methoxy-2,2,6,6-tetramethylpiperidin-N-oxid erfolgen.

Die anschließende Vinylierung mit 2 Mol eines Vinylmagnesiumsalzes erfolgt auf für Grignard-Reaktionen übliche Weise. Beispielsweise wird das Vinylmagnesiumhalogenid in einem Lösungsmittel, wie Tetrahydrofuran (THF) hergestellt und vorgelegt und hierzu langsam und unter Temperaturkontrolle eine Lösung des Adipindialdehyds in THF zugefügt. Durch übliche Aufarbeitung und fraktionierende Destillation erhält man das 1,9-Decadien-3,8-diol der Formel IX in recht guten Ausbeuten. Bezüglich näherer Einzelheiten dieses Reaktionsschrittes verweisen wir auf Bull. Soc. Chim. France 1959, Seiten 1248-1251, bes. 1248.

Die Carroll-Reaktion des 1,9-Decadien-3,8-diols mit einem Acetessigsäurealkylester, vorzugsweise mit Acetessigsäuremethylester erfolgt durch langsames Erhitzen beider Reaktionspartner auf Temperaturen von etwa 200°C. Dabei beginnt bei etwa 160°C die Abspaltung von Methanol und anschließend die $CO_2$-Abspaltung.

Bezüglich näherer Einzelheiten der Carroll-Reaktion, d.h. der Anlagerung $\alpha,\beta$-ungesättigter Alkohole an Verbindungen mit aktiver Methylengruppe verweisen wir auf Chem. Soc. Chim. France 1940, Seiten 704-706.

Die 2-fach ungesättigten neuen 2,15-Diketone der Formeln IIb bis IId können wie das bereits bekannte Diketon IIe auf einfache Weise und in guten Ausbeuten, beispielsweise durch katalytische Hydrierung an einem Palladiumkatalysator, in das gesättigte 2,15-Hexadecandion der Formel IIa überführt werden.

Zu 2:

Es wurde gefunden, daß man bei der intramolekularen Aldolkondensation des 2,15-Hexadecandions der Formel IIa Ausbeuten von bis zu 60 % der Theorie erhalten kann, wenn man diese Aldolkondensation nicht - wie bekannt - in stark verdünnter Lösung, sondern bei Temperaturen von 300 bis 450°C und in Gegenwart geringer Mengen Wasser in der Gasphase an einem Festbettkatalysator enthaltend als katalytisch wirksame Verbindung $TiO_2$, $CeO_2$ oder $ThO_2$, vorzugsweise $TiO_2$ durchführt. Es war sehr überraschend, daß diese lang untersuchte und sensible Umsetzung unter den drastischen Bedingungen einer Gasphasenreaktion so außerordentlich vorteilhaft verläuft.

Es war zwar schon eine Gasphasencyclisierungsreaktion, nämlich die Cyclisierung von Octadecandisäuredimethylester zu Cycloheptadecanon bekannt, jedoch wurde hierbei das gewünschte Cyclisierungsprodukt nur in Ausbeuten von 14 % der Theorie (gaschromatographisch ermittelt) erhalten (vgl. Parfumer and Flavorist, Vol. 7 (1982), Seite 57). Das Hauptprodukt dieser Cyclisierungsreaktion war polymeres Material.

Überraschenderweise wurde weiterhin gefunden, daß auch die 2-fach ungesättigten 2,15-Diketone der Formeln IIb bis IIe in der Gasphase an Katalysatoren enthaltend als katalytisch wirksame Verbindung $TiO_2$, $CeO_2$ oder $ThO_2$ mit guten Ausbeuten cyclisiert werden können.

Das war besonders überraschend, da aus J. Organometallic Chem. 264 (1984), Seiten 229-237, insbesondere 234, bekannt war, daß bei einem Versuch, das 2,15-Diketon der Formel IIe auf die bekannte Weise mit Diisobutylaluminiumhydrid, Phenol und Pyridin zu cyclisieren, nur Ausbeuten von etwa 6 % der Theorie an

dem entsprechenden cyclischen Keton erzielt werden konnten.

Gegenstand der Erfindung ist daher insbesondere ein Verfahren zur Herstellung von Muscon der Formel I

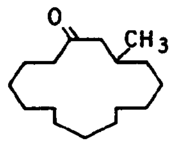

(I)

das dadurch gekennzeichnet ist, daß man offenkettige 2,15-Diketone der allgemeinen Formel II

$$CH_3\text{-}CO\text{-}X\text{-}CO\text{-}CH_3 \qquad (II)$$

in der X für einen der Reste

$$-(CH_2)_{12}- \qquad (a)$$

$$-CH{=}CH-(CH_2)_8-CH{=}CH- \qquad (b)$$

$$-CH_2-CH{=}CH-(CH_2)_6-CH{=}CH-CH_2- \qquad (c)$$

$$-CH_2-CH_2-CH{=}CH-(CH_2)_4-CH{=}CH-CH_2-CH_2- \qquad (d)$$

oder

$$-CH_2-CH_2-CH_2-CH{=}CH-(CH_2)_2-CH{=}CH-CH_2-CH_2-CH_2- \qquad (e)$$

steht,

bei Temperaturen von 300 bis 400°C, vorzugsweise 350 bis 380°C in Gegenwart von 5 bis 15 Gew.% Wasser, bezogen auf die Katalysatormenge, in der Gasphase an einem Festbettkatalysator enthaltend als katalytisch wirksame Verbindung $TiO_2$, $CeO_2$ oder $ThO_2$, vorzugsweise einen $TiO_2$-Katalysator, in Kontakt bringt und das sich hierbei durch intramolekulare Aldolkondensation bildende ungesättigte cyclische Keton katalytisch hydriert.

Mit besonderem Vorteil gelingt die erfindungsgemäße Aldolkondensation, wenn man die 2,15-Diketone der allgemeinen Formel II mit einem Alkali- oder Erdalkalimetalloxid dotierten $TiO_2$-Katalysator in Kontakt bringt.

Da sich die 2,15-Diketone der allgemeinen Formel II nach den oben beschriebenen Verfahren in technisch einfacher Weise herstellen lassen, ergibt sich somit ein technisch einfacher und vorteilhafter Syntheseweg zu dem begehrten Moschusriechstoff Muscon.

Die intramolekulare Aldolkondensation wird in der Gasphase bei einer Temperatur von 300 bis 400°C, vorzugsweise 350 bis 390°C, insbesondere 350 bis 380°C durchgeführt. Als Katalysatoren für das erfindungsgemäße Verfahren verwendet man mit besonderem Vorteil $TiO_2$, insbesondere mit Alkali- oder Erdalkalimetalloxiden dotiertes $TiO_2$, d.h. etwa 2 bis 10 Gew.-% $Na_2O$ und/oder $K_2O$ enthaltendes $TiO_2$. Die beschriebenen Katalysatoren können beispielsweise in Form von 2- bis 4-mm-Strängen oder in Form von Tabletten mit 3 bis 4 mm Durchmesser eingesetzt werden. Bei der Umsetzung in der Gasphase hält man zweckmäßig eine Katalysatorbelastung von 0,1 bis 30 g, insbesondere 1 bis 10 g, offenkettiges Diketon der Formel II pro g Katalysator und Stunde ein.

Zur Durchführung der Umsetzung wird im allgemeinen zunächst eine Lösung der kristallin anfallenden Diketone der Formel II in einem inerten organischen Lösungsmittel, wie Toluol oder einem Xylol, in einem Rohrreaktor verdampft und dann - gegebenenfalls zusammen mit einem Inertgas, wie Kohlendioxid oder Stickstoff - bei der gewünschten Reaktionstemperatur gasförmig über den fest angeordneten Katalysator geleitet. Um gute Ausbeuten an cyclischem Keton zu erzielen, ist weiterhin der Zusatz von geringen Mengen an Wasser notwendig, um eine mögliche Verkokung des Katalysators zu unterbinden. Vorteilhaft arbeitet man mit 5 bis 15 Gew.% Wasser, bezogen auf die Katalysatormenge.

Die Reaktionsprodukte werden mittels geeigneter Kühlvorrichtungen kondensiert und gaschromatographisch untersucht. Reaktionsprodukte mit noch unumgesetztem Ausgangsmaterial lassen sich gegebenenfalls erneut ohne weitere Reinigung direkt in die Cyclisierungsreaktion zurückführen.

Die bei dieser Aldolkondensation erhaltenen ungesättigten cyclischen Ketone der Formeln Xa bis Xc

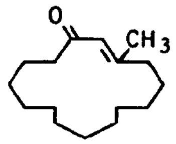

(Xa)

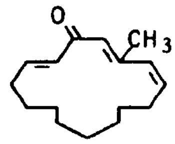

(Xb)

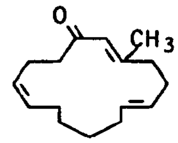

(Xc)

werden anschließend in an sich bekannter Weise durch Hydrieren in Gegenwart von Pd-Katalysatoren in den gewünschten Moschusriechstoff Muscon überführt.

Mit Hilfe der erfindungsgemäßen intramolekularen Aldolkondensation der 2,15-Diketone der Formel II und die anschließende katalytische Hydrierung kann der begehrte Moschusriechstoff Muscon in ausgezeichneten Ausbeuten erhalten werden. Durch Bereitstellen der neuen 2,15-Diketone der Formeln IIb bis IId sowie vorteilhaften Verfahren zu deren Herstellung ergibt sich somit ein technisch einfacher und vorteilhafter Syntheseweg von gut zugänglichen Ausgangsstoffen zu dem begehrten Muscon.

Beispiel 1

a) Herstellung von Adipindialdehyd (VII)

In einem 4-l-Dreihalskolben wurden bei Raumtemperatur (RT) die folgenden Chemikalien vorgelegt:

59 g (0,5 mol) 1,6-Hexandiol
3,9 g (0,025 mol) 2,2,6,6-Tetramethyl-piperidin-1-oxyd
3,0 g (0,025 mol) KBr
7,8 g (0,05 mol) $NaH_2PO_4$ x 2 $H_2O$
8,9 g (0,05 mol) $NaH_2PO_4$ x 2 $H_2O$
1 l $CH_2Cl_2$ + 375 ml Wasser.

Innerhalb von 2 Stunden (h) wurden anschließend 567 g (0,51 mol) einer ca. 13 %igen wäßrigen NaOCl-Lösung zugetropft (leicht exotherm). Dabei änderte sich der pH-Wert der Reaktionslösung von 6,7 nach 6,0. Nach dem Zutropfen wurde 15 Minuten (min) bei RT nachgerührt. Nach dem Abtrennen der organischen Phase wurde die wäßrige Phase 2mal mit je 200 ml $CH_2Cl_2$ extrahiert, die vereinigten organischen Phasen mit $NaHCO_3$-Lösung neutral gewaschen und mit $Na_2SO_4$ getrocknet. Nach dem Abfiltrieren von $Na_2SO_4$ wurde am Rotationsverdampfer das Lösungsmittel abdestilliert und vom Rückstand ein Gaschromatogramm aufgenommen.

Das Rohprodukt wurde direkt in der nächsten Stufe vinyliert.

b) Herstellung von 1,9-Decadien-3,8-diol (IX)

0,81 l einer 1,55 molaren Vinylmagnesiumchloridlösung in Tetrahydrofuran (THF) wurden vorgelegt. Innerhalb von 1 h wurden 48,0 g Adipindialdehyd (roh, 80,5 %ig), gelöst in 50 ml THF, unter Temperaturkontrolle zugetropft und anschließend 2 h nachgerührt. Nach der üblichen Aufarbeitung verblieben 59,6 g eines hellgelben Öls, das unter stark vermindertem Druck (Ölpumpe) fraktionierend destilliert wurde.

Die Ausbeute nach Destillation (Kp·0,5 = 108 bis 110°C) betrug 36,4 g (91 %ig), entsprechend einer Ausbeute von 64 % der Theorie.

c) Herstellung von 5,11-Hexadecadien-2,15-dion (IId)

Ein Gemisch aus 36,4 g eines 91 %igen (entsprechend 0,19 mol) 1,9-Decadien-3,8-diols und 74,5 g (0,61 mol) Acetessigsäuremethylester wurde innerhalb von 3 h auf 200°C erhitzt. Bei ca. 160°C begann die Methanolabspaltung, anschließend die $CO_2$-Abspaltung. Insgesamt destillierten 14,1 g Niedersieder (Sdp. <70°C) ab, und es wurden 9,5 l $CO_2$ (Theorie 10,2 l) abgespalten. Der Rückstand (73,5 g) wurde anschließend unter stark vermindertem Druck (Ölpumpe) fraktionierend destilliert.

Die Ausbeute nach Destillation (Kp·0,5 = 160 bis 170°C) betrug 38,6 g (81 %ig), entsprechend einer Ausbeute von 66 % der Theorie. Schmelzpunkt 54°C.

$^1$H-NMR ($CDCl_3$): δ = 1,32 (4H, m); 1,8-2,0 (4H, m); 2,1 (6H, s); 2,2-2,3 (4H, m); 2,5 (4H, b); 5,3-5,4 (4H, m); $^{13}$C-NMR ($CDCl_3$): δ = 26,9; 28,9; 29,6; 32,3; 43,6; 128,6; 131,3; 207,4.

d) Herstellung von Hexadecan-2,15-dion

38 g Hexadeca-5,11-dien-2,15-dion (0,152 mol) wurden in 200 ml Essigsäureethylester gelöst und bei 50°C unter Standardbedingungen mit 1,9 g 10 %ig Pd/C hydriert. Man erhielt nach Umkristallisation aus Petrolether 25,8 g eines 97 %igen Hexadecan-2,15-dions mit einem Schmelzpunkt von 80°C. Die Ausbeute betrug somit 66 % der Theorie.

Beispiel 2

a) Herstellung von 1,10-Decandial (IV)

Analog dem Verfahren gemäß Beispiel 1a) wurden 37 g (0,2 mol) 1,10-Decandiol durch Oxidation in 1,10-Decandial überführt. Ausbeute: 31 g (79 %ig) entsprechend 73 % der Theorie.

b) Herstellung von 3,13-Hexadecadien-2,15-dion (IIb) und 5,11-Hexadecadien-2,15-dion (IIc)

1150 ml einer 0,783 molaren Lösung von Triphenyl-phosphin-2-oxopropylen in Dichlormethan (0,9 mol) wurden bei RT vorgelegt. Zu dem so erhaltenen Wittigreagenz wurde innerhalb von 2 h eine Lösung von 69,5 g 1,10-Decandial (0,33 mol) in 250 ml Dichlormethan zugetropft (leicht exotherme Reaktion) und eine weitere Stunde nachgerührt. Zur Aufarbeitung wurde der Kolbeninhalt eingeengt und der Rückstand mit 1 l Cyclohexan ausgerührt. Das auskristallisierte Phosphinoxid wurde abfiltriert und die Mutterlauge nochmals mit 300 ml kaltem Cyclohexan gerührt. Nach dem Abtrennen der Restmenge des Phosphinoxids wurde die Mutterlauge eingeengt und unter vermindertem Druck (Ölpumpe) fraktionierend destilliert.

Die Ausbeute an 3,13-Hexadecadien-2,15-dion betrug 72 g (93 %ig) vom Sdp.: 136-139°C/0,03 mm, entsprechend einer Ausbeute von 81 % der Theorie.

$^{13}$C-NMR ($CDCl_3$): $\delta$ = 197,4 (C-2, C-15); 147,6 (C-4, C-13); 131,4 (C-3, C-14) ; 32,3 (C-5, C-12) ; 29,2 (C-8, C-9) ; 29,1 (C-7, C-10) ; 28,2 (C-6, C-11) ; 26,7 (C-1, C-16);

Die Auswertung der $^{13}$C-NMR-Daten der Reaktionsprodukte zeigte, daß bei der Destillation 25 % der erhaltenen Verbindung der Formel IIb in die Verbindung der Formel IIC isomerisiert sind.

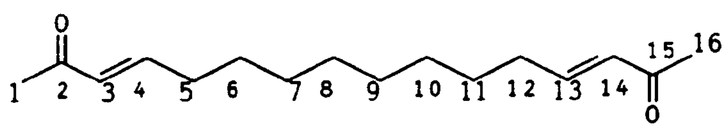

(IIb)

$^{13}$C-NMR ($CDCl_3$): $\delta$ = 206,0 (C-2, C-15); 134,9 (C-5); 122,3 (C-4); 47,5 (C-3); 32,4 (C-6); 28,6 (C-7);

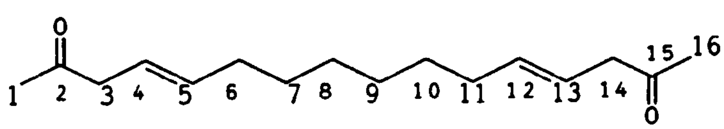

(IIc)

Beispiele 3 bis 8

Jeweils eine Lösung von 1,5 g eines gemäß Beispiel 1 erhaltenen Hexadecan-2,15-dions (IIa) in einem Gemisch aus 20 ml Dekalin und 10 ml Toluol wurde in einem Rohrreaktor verdampft und dann zusammen mit Stickstoff bei der in der Tabelle angegebenen Reaktionstemperatur gasförmig über einen in einer Säule (d = 22 mm; Länge = 50 cm) fest angeordneten Katalysator der aus der Tabelle ersichtlichen Zusammensetzung in Form von 2 bis 4 mm Strängen geleitet. In den Beispielen 1 bis 4 wurden dem Reaktionsgemisch außerdem die aus der Tabelle ersichtliche Menge Wasser zugesetzt.

Die Reaktionsprodukte wurden mittels einer geeigneten Kühlvorrichtung kondensiert, anschließend gaschromatographisch untersucht und dann in Gegenwart von Pd-Katalysatoren hydriert.

Die Reaktionsbedingungen sowie die erzielten Versuchsergebnisse sind in der folgenden Tabelle zusammengestellt.

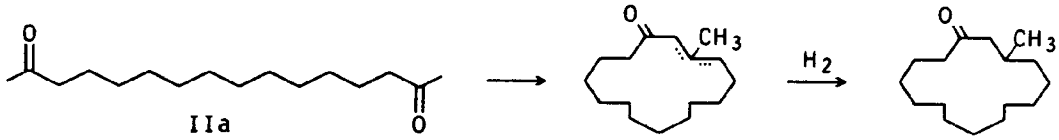


IIa

Tabelle

| Beispiel | Katalysator | Wasserzusatz [ml/h] | Reaktionstemperatur [°C] | LHSV [10 L $N_2$/h; 1,5 g IIa in 20 ml Dekalin + 10 ml Toluol/h] | Umsatz | Selektivität der Musconbildung |
|---|---|---|---|---|---|---|
| 3 | $TiO_2$ + 2 % $Na_2O$ | 5 | 350 | 0,01 | 51,3 | 70,5 |
| 4 | $TiO_2$ + 2 % $Na_2O$ | 5 | 370 | 0,01 | 62,6 | 86,7 |
| 5 | $TiO_2$ + 2 % $Na_2O$ | 15 | 380 | 0,01 | 53,4 | 49,6 |
| 6 | $TiO_2$ + 2 % $Na_2O$ | 15 | 400 | 0,01 | 96,6 | 19,3 |
| 7 | $TiO_2$ + 2 % $Na_2O$ | - | 370 | 0,01 | 100 | 7,8 |
| 8 | $TiO_2$ | 5 | 370 | 0,01 | 82,3 | 57,4 |

Beispiel 9

Eine Lösung von 4,5 g eines analog Beispiel 1a bis c hergestellten 5,11-Hexadecadien-2,15-dions IId in 20 ml Toluol pro Stunde (LHSV = 0,03) wurde unter Zusatz von 5 ml Wasser/h in einem Rohrreaktor verdampft und zusammen mit 10 L $N_2$/h und bei 380°C gasförmig über einen in einer Säule (d = 22 mm; 1 = 50 cm) fest angeordneten Katalysator bestehend aus $TiO_2$ und 2 % $Na_2O$ in Form von 2 bis 4 mm-Strängen geleitet. Die Reaktionsprodukte wurden mittels einer geeigneten Kühlvorrichtung kondensiert und anschließend gaschromatographisch untersucht.

Man erhielt bei einem Umsatz von 66 % eine Selektivität an Hexadehydromuscon von 63 % der Theorie.

## Patentansprüche

1. Verfahren zur Herstellung von Muscon der Formel I

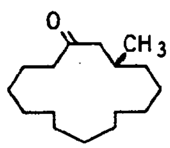

(I),

dadurch gekennzeichnet, daß man offenkettige 2,15-Diketone der allgemeinen Formel II

$CH_3$-CO-X-CO-$CH_3$ (II)

in der X für einen der Reste

$-(-CH_2-)_{12}$ (a)

$-CH=CH-(-CH_2-)_8$ CH= CH- (b)

$-CH_2-CH=CH-(-CH_2-)_6$ CH=CH-$CH_2$- (c)

$-CH_2-CH_2-CH=CH-(-CH_2-)_4$ CH=CH-$CH_2$-$CH_2$- (d)

oder

$-CH_2-CH_2-CH_2-CH=CH-(-CH_2-)_2$ CH=CH-$CH_2$-$CH_2$-$CH_2$- (e)

steht,

bei Temperaturen von 300 bis 400°C in Gegenwart von 5 bis 15 Gew.% Wasser, bezogen auf die Katalysatormenge, in der Gasphase an einem Festbettkatalysator enthaltend als katalytisch wirksame Verbindung $TiO_2$, $CeO_2$ oder $ThO_2$ in Kontakt bringt, und das sich hierbei durch intramolekulare Aldolkondensation bildende ungesättigte cyclische Keton katalytisch hydriert.

2. Verfahren zur Herstellung von Muscon gemäß Anspruch 1, dadurch gekennzeichnet, daß man die 2,15-Diketone der allgemeinen Formel II bei Temperaturen von 350 bis 380°C mit dem Katalysator in Kontakt bringt.

3. Verfahren zur Herstellung von Muscon gemäß Anspruch 1, dadurch gekennzeichnet, daß man die 2,15-Diketone der allgemeinen Formel II mit einem $TiO_2$-Katalysator in Kontakt bringt.

4. Verfahren zur Herstellung von Muscon gemäß Anspruch 1, dadurch gekennzeichnet, daß man die 2,15-Diketone der allgemeinen Formel II mit einem mit Alkali- oder Erdalkalimetalloxiden dotierten $TiO_2$-Katalysator in Kontakt bringt.

5. Offenkettige 2-fach ungesättigte 2,15-Diketone der allgemeinen Formel II

$CH_3$-CO-X-CO-$CH_3$ (II),

in der X für einen unverzweigten Alkadienylenrest der Struktur

$-CH=CH-(-CH_2-)_8$ CH=CH- (b),

$$-CH_2-CH=CH-(-CH_2-)_6-CH=CH-CH_2- \quad (c)$$

oder

$$CH_2-CH_2-CH=CH-(-CH_2-)_4-CH=CH-CH_2-CH_2- \quad (d)$$

steht.

6. Verfahren zur Herstellung eines Gemisches der 2-fach ungesättigten 2,15-Diketone der Formeln IIb und IIc gemäß Anspruch 5

$$CH_3-CO-CH=CH-(-CH_2-)_8-CH=CH-CO-CH_3 \quad (IIb)$$

$$CH_3-CO-CH_2-CH=CH-(-CH_2-)_6-CH=CH-CH_2-CO-CH_3 \quad (IIc),$$

dadurch gekennzeichnet, daß man 1,10-Decan-diol der Formel III

$$OH-CH_2-(-CH_2-)_8-CH_2-OH \quad (III)$$

oxidativ dehydriert,
das dabei erhaltene 1,10-Decan-dial der Formel IV

$$OHC-(-CH_2-)_8-CHO \quad (IV)$$

einer Wittig-Reaktion mit 2 Mol eines 2-Oxo-propenyl-triphenylphosphoniumsalzes der Formel V

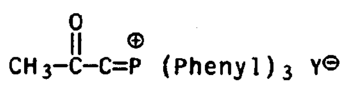

(V),

worin
$Y^{\ominus}$ für $Cl^-$, $Br^-$ oder $HSO_4^{\ominus}$ steht, unterwirft und das Reaktionsprodukt destilliert.

7. Verfahren zur Herstellung des 2-fach ungesättigten 2,15-Diketons der Formel IId gemäß Anspruch 5

$$CH_3-CO-CH_2-CH_2-CH=CH-(-CH_2-)_4-CH=CH-CH_2-CH_2-CO-CH_3 \quad (IId),$$

dadurch gekennzeichnet, daß man
A. 1,6-Hexan-diol der Formel VI

$$HO-CH_2-(-CH_2-)_4-CH_2-OH \quad (VI)$$

oxidativ dehydriert,
B. den dabei erhaltenen Adipindialdehyd der Formel VII

$$OHC-(-CH_2-)_4-CHO \quad (VII)$$

mit je 2 Mol eines Vinylmagnesiumhalogenids der Formel VIII

$$CH_2=CH-Mg-Hal \quad (VIII),$$

in der Hal für Cl oder Br steht, in einer Grignard-Reaktion umsetzt und
C. das dabei erhaltene 1,9-Decadien-3,8-diol der Formel IX

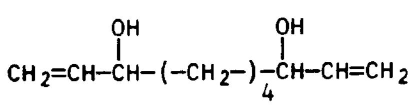

(IX)

einer Carroll-Reaktion mit einem Acetessigsäurealkylester unterwirft.

8. Verfahren zur Herstellung von Muscon gemäß Anspruch 1, dadurch gekennzeichnet, daß man ein offenkettiges 2,15-Diketon der Formeln IIa oder IId

$$CH_3-CO-(-CH_2-)_{12}-CO-CH_3 \quad (IIa)$$

$$CH_3-CO-CH_2-CH_2-CH=CH-(-CH_2-)_4-CH=CH=CH_2-CH_2-CO-CH_3 \quad (IId)$$

mit dem Katalysator in Kontakt bringt, das auf folgende Weise hergestellt wurde:

A. Oxidatives Dehydrieren von 1,6-Hexan-diol der Formel VI

$$HO-CH_2-(-CH_2-)_4-CH_2-OH \quad (VI)$$

B. Grignard-Reaktion des dabei erhaltenen Adipindialdehyds der Formel VII

$$OHC-(-CH_2-)_4-CHO \quad (VII)$$

mit je 2 Mol eines Vinylmagnesiumhalogenids der Formel VIII

$$CH_2=CH-Mg-Hal \quad (VIII),$$

worin Hal für Cl oder Br steht,

C. Carroll-Reaktion des dabei erhaltenen 1,9-Decadien-3,8-diols der Formel IX

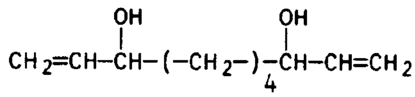

(IX)

mit einem Acetessigsäurealkylester und

D. ggf. anschließende katalytische Hydrierung des dabei erhaltenen offenkettigen Ketons der Formel IId.

**9.** Verfahren zur Herstellung von Muscon gemäß Anspruch 1, dadurch gekennzeichnet, daß man ein offenkettiges 2,15-Diketon der Formeln IIa oder ein Gemisch der 2,15-Diketone der Formeln IIb und IIc

$$CH_3-CO-(-CH_2-)_{12}CO-CH_3 \quad IIa$$

$$CH_3-CO-CH=CH-(-CH_2-)_8-CH=CH-CO-CH_3 \quad IIb$$

$$CH_3-CO-CH_2-CH=CH-(-CH_2-)_6-CH=CH-CH_2-CO-CH_3 \quad IIc$$

mit dem Katalysator in Kontakt bringt, das auf folgende Weise hergestellt wurde:

A. Oxidatives Dehydrieren von 1,10-Decandiol der Formel III

$$OH-CH_2-(-CH_2-)_8-CH_2-OH \quad (III),$$

B. Wittig-Reaktion des dabei erhaltenen 1,10-Decandial der Formel IV

$$OHC-(-CH_2-)_8-CHO \quad (IV)$$

mit je 2 Mol eines 2-Oxo-propenyl-triphenylphosphoniumsalzes der Formel V

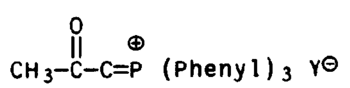

(V),

$Y^{\ominus}$ für $Cl^-$, $Br^-$ oder $HSO_4^{\ominus}$ steht, und

C. ggf. anschließende katalytische Hydrierung des dabei erhaltenen Gemisches der 2,5-Diketone der Formeln IIb und IIc.

**10.** Verwendung der offenkettigen 2-fach ungesättigten 2,15-Diketone der allgemeinen Formeln IIb, IIc und IId gemäß Anspruch 5

$$CH_3-CO-CH=CH-(-CH_2-)_8-CH=CH-CO-CH_3 \quad (IIb)$$

$$CH_3-CO-CH_2-CH=CH-(-CH_2-)_6-CH=CH-CH_2-CO-CH_3 \quad (IIc),$$

$$CH_3\text{-}CO\text{-}CH_2\text{-}CH_2\text{-}CH{=}CH\text{-}(\text{-}CH_2\text{-})_4\text{-}CH{=}CH\text{-}CH_2\text{-}CH_2\text{-}CO\text{-}CH_3 \qquad \text{(IId)}$$

als Zwischenprodukte zur Herstellung von Muscon der Formel I

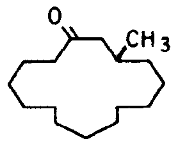

(I)

## Claims

1. A process for the preparation of muscone of the formula I

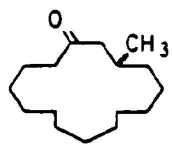

(I)

wherein an open-chain 2,15-diketone of the formula II

$$CH_3\text{-}CO\text{-}X\text{-}CO\text{-}CH_3 \qquad \text{(II)}$$

where X is one of the radicals

$$\text{-}(\text{-}CH_2\text{-})_{12}\text{-} \qquad \text{(a)}$$

$$\text{-}CH{=}CH\text{-}(\text{-}CH_2\text{-})_8\text{-}CH{=}CH\text{-} \qquad \text{(b)}$$

$$\text{-}CH_2\text{-}CH{=}CH\text{-}(\text{-}CH_2\text{-})_6\text{-}CH{=}CH\text{-}CH_2\text{-} \qquad \text{(c)}$$

$$\text{-}CH_2\text{-}CH_2\text{-}CH{=}CH\text{-}(\text{-}CH_2\text{-})_4\text{-}CH{=}CH\text{-}CH_2\text{-}CH_2\text{-} \qquad \text{(d)}$$

or

$$\text{-}CH_2\text{-}CH_2\text{-}CH_2\text{-}CH{=}CH\text{-}(\text{-}CH_2\text{-})_2\text{-}CH{=}CH\text{-}CH_2\text{-}CH_2\text{-}CH_2\text{-} \qquad \text{(e)}$$

is brought into contact, at from 300 to 400°C in the presence of from 5 to 15% by weight, based on the amount of catalyst, of water, in the gas phase, with a fixed-bed catalyst containing $TiO_2$, $CeO_2$ or $ThO_2$ as the catalytically active compound and the unsaturated cyclic ketone formed by intramolecular aldol condensation is subjected to catalytic hydrogenation.

2. A process for the preparation of muscone as claimed in claim 1, wherein a 2,15-diketone of the formula II is brought into contact with the catalyst at from 350 to 380°C.

3. A process for the preparation of muscone as claimed in claim 1, wherein a 2,15-diketone of the formula II is brought into contact with a $TiO_2$ catalyst.

4. A process for the preparation of muscone as claimed in claim 1, wherein a 2,15-diketone of the formula II is brought into contact with a $TiO_2$ catalyst doped with alkali metal oxides or alkaline earth metal oxides.

5. An open-chain diunsaturated 2,15-diketone of the formula II

$$CH_3\text{-}CO\text{-}X\text{-}CO\text{-}CH_3 \qquad \text{(II)}$$

where X is a straight-chain alkadienylene radical of the structure

$$\text{-}CH{=}CH\text{-}(\text{-}CH_2\text{-})_8\text{-}CH{=}CH\text{-} \qquad \text{(b)}$$

$$\text{-}CH_2\text{-}CH{=}CH\text{-}(\text{-}CH_2\text{-})_6\text{-}CH{=}CH\text{-}CH_2\text{-} \qquad \text{(c)}$$

or

$$-CH_2-CH_2-CH=CH-(-CH_2-)_4-CH=CH-CH_2-CH_2- \quad (d).$$

6. A process for the preparation of a mixture of the diunsaturated 2,15-diketones of the formulae IIb and IIc as claimed in claim 5

$$CH_3-CO-CH=CH-(-CH_2-)_8-CH=CH-CO-CH_3 \quad (IIb)$$

$$CH_3-CO-CH_2-CH=CH-(-CH_2-)_6-CH=CH-CH_2-CO-CH_3 \quad (IIc)$$

wherein 1,10-decanediol of the formula III

$$OH-CH_2-(-CH_2-)_8-CH_2-OH \quad (III)$$

is dehydrogenated under oxidative conditions, the resulting 1,10-decanedial of the formula IV

$$OHC-(-CH_2-)_8-CHO \quad (IV)$$

is subjected to a Wittig reaction with 2 moles of a 2-oxopropenyltriphenylphosphonium salt of the formula V

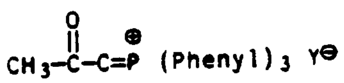

(V)

where $Y^{\ominus}$ is $Cl^-$, $Br^-$ or $HSO_4^{\ominus}$, and the reaction product is distilled.

7. A process for the preparation of the diunsaturated 2,15-diketone of the formula IId as claimed in claim 5

$$CH_3-CO-CH_2-CH_2-CH=CH-(-CH_2-)_4-CH=CH-CH_2-CH_2-CO-CH_3 \quad (IId)$$

wherein

A. 1,6-hexanediol of the formula VI

$$HO-CH_2-(-CH_2-)_4-CH_2-OH \quad (VI)$$

is dehydrogenated under oxidative conditions,

B. the resulting adipodialdehyde of the formula VII

$$OHC-(-CH_2-)_4-CHO \quad (VII)$$

is reacted with 2 moles of a vinylmagnesium halide of the formula VIII

$$CH_2=CH-Mg-Hal \quad (VIII)$$

where Hal is Cl or Br, in a Grignard reaction, and

C. the resulting 1,9-decadiene-3,8-diol of the formula IX

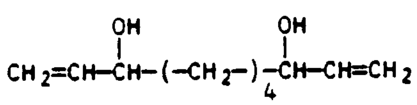

(IX)

is subjected to a Carroll reaction with an alkyl acetoacetate.

8. A process for the preparation of muscone as claimed in claim 1, wherein an open-chain 2,15-diketone of the formula IIa or IId

$$CH_3-CO-(-CH_2-)_{12}-CO-CH_3 \quad (IIa)$$

$$CH_3-CO-CH_2-CH_2-CH=CH-(-CH_2-)_4-CH=CH-CH_2-CH_2-CO-CH_3 \quad (IId)$$

is brought into contact with the catalyst which has been prepared in the following manner:

A. Oxidative dehydrogenation of 1,6-hexanediol of the formula VI

$$HO-CH_2-(-CH_2-)_4-CH_2-OH \quad (VI)$$

B. Grignard reaction of the resulting adipodialdehyde of the formula VII

$OHC\text{-}(\text{-}CH_2\text{-})_4\text{-}CHO$ (VII)

with 2 moles of a vinylmagnesium halide of the formula VIII

$CH_2=CH\text{-}Mg\text{-}Hal$ (VIII)

where Hal is Cl or Br,

C. Carroll reaction of the resulting 1,9-decadiene-3,8-diol of the formula IX

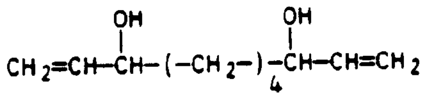

(IX)

with an alkyl acetoacetate, and

D. if necessary, subsequent catalytic hydrogenation of the resulting open-chain ketone of the formula IId.

**9.** A process for the preparation of muscone as claimed in claim 1, wherein an open-chain 2,15-diketone of the formula IIa or a mixture of the 2,15-diketones of the formulae IIb and IIc

$CH_3\text{-}CO\text{-}(\text{-}CH_2\text{-})_{12}\text{-}CO\text{-}CH_3$ IIa

$CH_3\text{-}CO\text{-}CH=CH\text{-}(\text{-}CH_2\text{-})_8\text{-}CH=CH\text{-}CO\text{-}CH_3$ IIb

$CH_3\text{-}CO\text{-}CH_2\text{-}CH=CH\text{-}(\text{-}CH_2\text{-})_6\text{-}CH=CH\text{-}CH_2\text{-}CO\text{-}CH_3$ IIc

is brought into contact with the catalyst which has been prepared in the following manner:

A. Oxidative dehydrogenation of 1,10-decanediol of the formula III

$OH\text{-}CH_2\text{-}(\text{-}CH_2\text{-})_8\text{-}CH_2\text{-}OH$ (III)

B. Wittig reaction of the resulting 1,10-decanedial of the formula IV

$OHC\text{-}(\text{-}CH_2\text{-})_8\text{-}CHO$ (IV)

with 2 moles of a 2-oxopropenyltriphenylphosphonium salt of the formula V

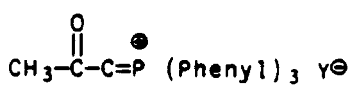

(V)

where $Y^{\ominus}$ is $Cl^-$, $Br^-$ or $HSO_4^{\ominus}$, and

C. if necessary, subsequent catalytic hydrogenation of the resulting mixture of the 2,5-diketones of the formulae IIb and IIc.

**10.** Use of an open-chain diunsaturated 2,15-diketone of the formulae IIb, IIc and IId as claimed in claim 5

$CH_3\text{-}CO\text{-}CH=CH\text{-}(\text{-}CH_2\text{-})_8\text{-}CH=CH\text{-}CO\text{-}CH_3$ (IIb)

$CH_3\text{-}CO\text{-}CH_2\text{-}CH=CH\text{-}(\text{-}CH_2\text{-})_6\text{-}CH=CH\text{-}CH_2\text{-}CO\text{-}CH_3$ (IIc)

$CH_3\text{-}CO\text{-}CH_2\text{-}CH_2\text{-}CH=CH\text{-}(\text{-}CH_2\text{-})_4\text{-}CH=CH\text{-}CH_2\text{-}CH_2\text{-}CO\text{-}CH_3$ (IId)

as an intermediate for the preparation of muscone of the formula I

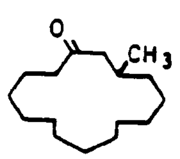

(I)

## Revendications

1. Procédé de préparatian de muscone de formule I

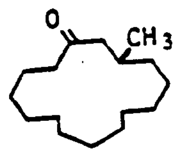

(I),

caractérisé en ce qu'on met des 2,15-dicétones en chaîne ouverte de formule générale II

$CH_3$-CO-X-CO-$CH_3$ (II)

dans laquelle X est mis pour l'un des restes suivants

-(-$CH_2$-)$_{12}$- (a)

-CH=CH-(-$CH_2$-)$_8$-CH=CH- (b)

$CH_2$-CH=CH-(-$CH_2$-)$_6$-CH=CH-$CH_2$- (c)

-$CH_2$-$CH_2$-CH=CH-(-$CH_2$-)$_4$-CH=CH-$CH_2$-$CH_2$- (d)

ou

-$CH_2$-$CH_2$-$CH_2$-CH=CH-(-$CH_2$-)$_2$-CH=CH-$CH_2$-$CH_2$-$CH_2$- (e)

en contact en phase gazeuse avec un catalyseur en lit fixe contenant $TiO_2$, $CeO_2$ ou $ThO_2$ comme composé à activité catalytique, à des températures de 300 à 400°C et en présence de 5 à 15% en poids d'eau par rapport à la quantité de catalyseur, et on soumet à une hydrogénation catalytique la cétone cyclique insaturée qui se forme dans ces conditions par aldolisation intramoléculaire.

2. Procédé de préparatian de muscone selon la revendication 1, caractérisé en ce qu'on met les 2,15-dicétones de formule générale II en contact avec le catalyseur à des températures de 350 à 380°C.

3. Procédé de préparation de muscone selon la revendication 1, caractérisé en ce qu'on met les 2,15-dicétones de formule générale II en contact avec un catalyseur à base de $TiO_2$.

4. Procédé de préparation de muscone selon la revendication 1, caractérisé en ce qu'on met les 2,15-dicétones de formule générale II en contact avec un catalyseur à base de $TiO_2$ dopé avec des oxydes de métaux alcalins au alcalino-terreux.

5. 2,15-Dicétones en chaîne ouverte à double insaturation de formule générale II

$CH_3$-CO-X-CO-$CH_3$ (II)

dans laquelle X est mis pour un reste alcadiényle non ramifié de structure

-CH=CH-(-$CH_2$-)$_8$-CH=CH- (b)

-$CH_2$-CH=CH-(-$CH_2$-)$_6$-CH=CH-$CH_2$- (c)

ou

-$CH_2$-$CH_2$-CH=CH-(-$CH_2$)$_4$-CH=CH-$CH_2$-$CH_2$- (d)

6. Procédé de préparation d'un mélange des 2,15-dicétones à double insaturation de formules IIb et IIc selon la revendication 5

$CH_3$-CO-CH=CH-(-$CH_2$-)$_8$-CH=CH-CO-$CH_3$ (IIb)

$CH_3$-CO-$CH_2$-CH=CH-($CH_2$-)$_6$-CH=CH-$CH_2$-CO-$CH_3$ (IIc)

caractérisé en ce qu'an déshydragèné par oxydation du 1,10-décanediol de formule III

OH-$CH_2$-(-$CH_2$-)$_8$-$CH_2$-OH (III)

on soumet le 1,10-décanedial de formule IV ainsi obtenu

OHC-(-$CH_2$-)$_8$-CHO (IV)

à une réaction de Wittig avec 2 moles d'un sel de 2-oxopropényltriphénylphosphonium de formule V

$CH_3-\overset{O}{\overset{\|}{C}}-C=\overset{+}{P}$ (phényle)$_3$ $Y^-$ (V),

dans laquelle $Y^-$ est mis pour $Cl^-$, $Br^-$ ou $HSO_4^-$, et on distille le produit de la réaction.

**7.** Procédé de préparation de la 2,15-dicétone à double insaturation de formule IId selon la revendication 5

$CH_3-CO-CH_2-CH_2-CH=CH-(-CH_2-)_4-CH=CH-CH_2-CH_2-CO-CH_3$ (IId)

caractérisé en ce que

A. on déshydrogène par oxydation du 1,6-hexanediol de formule VI

$HO-CH_2-(-CH_2-)_4-CH_2-OH$ (VI)

B. on fait réagir, en une réaction de Grignard, le dialdéhyde adipique de formule VII ainsi obtenu

$OHC-(-CH_2-)_4-CHO$ (VII)

avec, chaque fois, 2 moles d'un halogénure de vinylmagnésium de formule VIII

$CH_2=CH-Mg-Hal$ (VIII)

dans laquelle Hal est mis pour Cl ou Br, et

C. on soumet le 1,9-décadiène-3,8-diol de formule IX ainsi obtenu

```
           OH              OH
           '               '
CH2=CH-CH-(-CH2-)4-CH-CH=CH2        (IX)
```

à une réaction de Carroll avec un acétylacétate d'alkyle.

**8.** Procédé de préparation de muscone de formule I, caractérisé en ce qu'on met en contact avec le catalyseur une 2,15-dicétone en chaîne ouverte de formule IIa au IId

$CH_3-CO-(-CH_2-)_{12}-CO-CH_3$ (IIa)

$CH_3-CO-CH_2-CH_2-CH=CH-(-CH_2)_4-CH=CH-CH_2-CH_2-CO-CH_3$ (IId)

qui a été préparée de la manière suivante:

A. Déshydrogénation par oxydation du 1,6-hexanediol de formule VI

$HO-CH_2-(-CH_2-)_4-CH_2-OH$ (VI)

B. Réaction de Grignard du dialdéhyde adipique de formule VII ainsi obtenu

$OHC-(-CH_2-)_4-CHO$ (VII)

avec, chaque fois, 2 moles d'un halogénure de vinylmagnésium de formule VIII

$CH_2=CH-Mg-Hal$ (VIII)

dans laquelle Hal est mis pour Cl ou Br,

C. Réaction de Carroll du 1,9-décadiène-3,8-diol de formule IX ainsi obtenu

```
           OH              OH
           '               '
CH2=CH-CH-(-CH2-)4-CH-CH=CH2        (IX)
```

avec un acétylacétate d'alkyle et

D. éventuellement, hydrogénation catalytique subséquente de la cétone en chaîne ouverte de formule IId ainsi obtenue.

**9.** Procédé de préparation de muscone selon la revendication 1, caractérisé en ce qu'on met en contact avec le catalyseur une 2,15-dicétone en chaîne ouverte de formule IIa ou un mélange des 2,15-dicétones de formules IIb et IIc

$CH_3-CO-(-CH_2-)_{12}-CO-CH_3$ (IIa)

$CH_3-CO-CH=CH-(-CH_2-)_8-CH=CH-CO-CH_3$ (IIb)

$CH_3-CO-CH_2-CH=CH-(-CH_2-)_6-CH=CH-CH_2-CO-CH_3$ (IIc)

qui a été préparé de la manière suivante:

A. Déshydrogénation par oxydation du 1,10-décanediol de formule III

$OH-CH_2-(-CH_2-)_8-CH_2-OH$ (III)

B. Réaction de Wittig du 1,10-décanedial de formule IV ainsi obtenu

$OHC-(-CH_2-)_8-CHO$ (IV)

avec, chaque fois, 2 moles d'un sel de 2-oxopropényltriphénylphosphonium de formule V

```
     O    +
     ''
CH3-C-C=P (phényle)3 Y-        (V),
```

dans laquelle $Y^-$ est mis pour $Cl^-$, $Br^-$ ou $HSO_4^-$, et
C. éventuellement, hydrogénation catalytique subséquente du mélange ainsi obtenu des 2,5-dicétones de formules IIb et IIc.

**10.** Utilisation des 2,15-dicétones en chaîne ouverte à double insaturation de formules générales IIb, IIc et IId selon la revendication 5

$$CH_3\text{-}CO\text{-}CH{=}CH\text{-}(\text{-}CH_2\text{-})_8\text{-}CH{=}CH\text{-}CO\text{-}CH_3 \quad (IIb)$$

$$CH_3\text{-}CO\text{-}CH_2\text{-}CH{=}CH\text{-}(CH_2\text{-})_6\text{-}CH{=}CH\text{-}CH_2\text{-}CO\text{-}CH_3 \quad (IIc)$$

$$CH_3\text{-}CO\text{-}CH_2\text{-}CH_2\text{-}CH{=}CH\text{-}(\text{-}CH_2\text{-})_4\text{-}CH{=}CH\text{-}CH_2\text{-}CH_2\text{-}CO\text{-}CH_3 \quad (IId)$$

comme produits intermédiaires pour la préparation de muscone de formule I

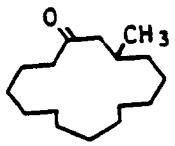

(I)